# EUROPÄISCHE PATENTANMELDUNG

(11) **EP 2 446 863 A1**
(43) Veröffentlichungstag der Anmeldung: **02.05.2012**
(21) Anmeldenummer: 11184599.6
(22) Anmeldetag: 11.10.2011
(51) Int. Cl.: A61F 2/90

(54) **Stent mit radial asymmetrischer Kraftverteilung**

(30) Priorität: 29.10.2010 US 407922 P
(71) Anmelder: Biotronik AG, 8180 Bülach (CH)
(72) Erfinder: Ulmer, Jens, 8008 Zürich (CH)
(74) Vertreter: Lindner-Vogt, Karin L.

(57) **Zusammenfassung**

Die vorliegende Erfindung betrifft einen expandierbarer Stent umfassend einen röhrenförmigen Grundkörper mit einem Lumen entlang einer longitudinalen Achse, dadurch gekennzeichnet, dass der Grundkörper mindestens einen radial asymmetrisch expandierbaren Abschnitt (2) aufweist, wobei der radial asymmetrisch expandierbare Abschnitt eine Dehnfähigkeit aufweist, die im Vergleich zur durchschnittlichen Dehnfähigkeit im übrigen Grundkörper des Stents erhöht ist.

## Beschreibung

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu einen Grundkörper auf, der eine Vielzahl von umlaufenden Stützstrukturen z. B. aus metallischen Streben aufweist, der zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents weisen einen röhrenförmigen Grundkörper auf, der ein Lumen entlang einer longitudinalen Achse umfasst. Der Grundkörper weist eine Mehrzahl von umlaufenden Stützstrukturen auf, z. B. umlaufende zylindrische Mäanderringe oder Helices, die nacheinander folgend entlang der longitudinalen Achse angeordnet sind. Diese Stützstrukturen sind jeweils aus einer Aneinanderreihung von Diagonalelementen und Bogenelementen (auch Kronen genannt) aufgebaut und bilden die geometrische Grundeinheit von modernen Stentdesigns. Die Stützstrukturen werden durch Verbindungselemente - sogenannte Konnektoren - in longitudinaler Richtung miteinander verbunden. Einerseits müssen diese Konnektoren so angeordnet sein, dass sie eine ausreichende Biegeflexibilität des Stents gewährleisten, andererseits dürfen sie einen Crimp- und/oder Dilatationsprozess nicht behindern.

Die Stents des Standes der Technik zeichnen sich dadurch aus, dass sie bei Expansion eine achsensymmetrische Radialkraftverteilung aufweisen. Dadurch ist die radiale Kraft, die auf die Gefäßwände ausgeübt wird, entlang des Umfangs des Stents im Wesentlichen gleich verteilt. Die meisten zu behandelnden Stenosen weisen allerdings selbst keine radialsymmetrische Ausdehnung, bzw. Festigkeitsprofil innerhalb des Gefäßes auf (sog. exzentrische Stenose). Dies hat zur Folge, dass bei Behandlung solcher exzentrischen Stenosen mittels handelsüblicher Stents umliegendes gesundes Gewebe unnötig belastet wird und es dort während der Behandlung zu Verletzungen der Gefäßwand kommen kann. Solche traumatologischen Gefäßwandveränderungen, auch und gerade in gesundem Gewebe, werden heutzutage als wesentlicher Auslöser einer Gewebeneubildung (neointimale Hyperplasie) und damit verbunden mit der Gefahr einer Restenosebildung betrachtet.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung Mittel bereitzustellen, die die Behandlung von Stenosen erlauben, aber umliegendes gesundes Gewebe weniger stark belasten.

Die vorliegende Erfindung löst diese Aufgabe durch Bereitstellung eines expandierbaren Stents umfassend einen röhrenförmigen Grundkörper mit einem Lumen entlang einer longitudinalen Achse, dadurch gekennzeichnet, dass der Grundkörper mindestens einen radial asymmetrisch expandierbaren Abschnitt aufweist, wobei der radial asymmetrisch expandierbare Abschnitt eine durchschnittliche Dehnfähigkeit aufweist, die im Vergleich zur durchschnittlichen Dehnfähigkeit im übrigen Grundkörper des Stents erhöht ist.

Bevorzugt beträgt das Verhältnis δ der durchschnittlichen Dehnfähigkeit im übrigen Grundkörper des Stents zur durchschnittlichen Dehnfähigkeit im radial asymmetrisch expandierbaren Abschnitt 1 > δ ≧0,01, bevorzugt 0,95 > ≥ δ ≥ 0,1 und besonders bevorzugt 0,9 ≧ δ ≧ 0,7.

Der erfindungsgemäße Stent zeichnet sich dadurch aus, dass die auf die Gefäßwand wirkenden Radialkräfte während der Expansion asymmetrisch verteilt sind. Bereiche mit einer erhöhten Dehnfähigkeit dehnen sich während der Expansion stärker aus als die übrigen Bereiche des Grundkörpers zu deren Dehnung eine vergleichsweise erhöhte Kraft aufgewendet werden muss (geringere Dehnfähigkeit). Dadurch wird erreicht, dass durch anlegen einer einheitlichen radialsymmetrischen Kraft (etwa in Form des Inflationsdrucks durch den Dilatationsballon), sich der Stent in Bereichen mit erhöhter Dehnfähigkeit weiter ausdehnt als in Bereichen mit relativ dazu geringerer Dehnfähigkeit. Der erfindungsgemäße Stent erlaubt es nun in besonders vorteilhafter Weise exzentrische Gefäßengstellen zu behandeln, wobei bevorzugt die erkrankten Bereiche der Gefäßwand durch die erhöhte Dehnbarkeit des asymmetrisch dilatierbaren Stents, aufgeweitet werden, während das Gewebe in den umliegenden gesunden Teilen der Gefäßwand nicht so stark gedehnt werden.. So kann eine Stenose effektiv beseitigt und der Blutfluss wieder hergestellt werden, im Wesentlichen ohne das umliegende gesunde Gefäßgewebe zu beschädigen. Durch Verwendung des erfindungsgemäßen Stents wird somit ohne Einsatz von Medikamenten oder Wirkstoffen die Gefahr von unerwünschter Gewebeneubildung (neointimale Hyperplasie) verringert und damit einhergehend die Wahrscheinlichkeit des Auftretens von Restenosen verkleinert.

Der erfindungsgemäße expandierbare Stent weist einen röhrenförmigen Grundkörper auf, der ein Lumen entlang einer longitudinalen Achse umschließt. Durch dieses Lumen kann, nach erfolgter Applikation des Stents in ein Blutgefäß, ein Blutfluss erfolgen. Der Grundkörper umfasst eine Mehrzahl von umlaufenden Stützstrukturen, die nacheinander folgend entlang der longitudinalen Achse angeordnet sind und das Lumen umschließen.

Die Stützstrukturen sind jeweils aus einer Aneinanderreihung von Diagonalelementen und Bogenelementen aufgebaut. Die Diagonalelemente weisen eine langgestreckte Form mit zwei Enden auf und verbinden zwei Bogenelemente mit gegenläufig ausgerichteter Krümmung. Die Diagonalelemente sind im Wesentlichen für die Erstreckung der Stützstruktur in longitudinaler Achsenrichtung verantwortlich. Die Bogenelemente sind gekrümmt und verbinden zwei aufeinander folgende Diagonalelemente einer Stützstruktur derart miteinander, dass diese entlang einer zur longitudinalen Achse vertikal verlaufenden Achse übereinander zu liegen kommen, wobei eine ringförmige umlaufende Struktur entsteht, die ein Lumen umschließt.

Der Grundkörper umfasst neben einer Mehrzahl von Stützstrukturen einen oder mehrere Konnektoren, wobei zwei aufeinander folgende umlaufende Stützstrukturen über mindestens einen Konnektor miteinander verbunden sind. Die Konnektoren des erfindungsgemäßen Stents sind bevorzugt derart ausgestaltet, dass eine Mehrzahl von Stützstrukturen zu einem Grundkörper verbunden werden können, der für den Einsatz in einem expandierbaren Stent geeignet ist. Dazu ist jeweils ein Konnektor an einem ersten Ende mit einem Diagonalelement oder einem Bogenelement einer ersten Stützstruktur verbunden und an einem zweiten Ende mit einem Diagonalelement oder einem Bogenelement einer zweiten Stützstruktur. Zwei aufeinander folgende Stützstrukturen können auch über mehr als einen Konnektor miteinander verbunden sein. Einer, mehrere oder alle Konnektoren des erfindungsgemäßen Stents können eine im Wesentlichen langgestreckte Form mit zwei entgegengesetzten Enden aufweisen. Bevorzugt sind die Konnektoren so lang, dass eine ausreichende Flexibilität der beiden benachbarten Stützstrukturen gewährleistet ist, aber nicht so lang, dass der erfindungsgemäße Stent torsionsweich wird. Einer, mehrere oder alle Konnektoren eines erfindungsgemäßen Stents können eine mehr oder weniger geschwungene Form aufweisen. Die Konnektoren sind in grundsätzlich longitudinaler Richtung zwischen den beiden zu verbindenden umlaufenden Stützstrukturen ausgerichtet, wobei die Konnektoren nicht notwendigerweise in paralleler Ausrichtung zur longitudinalen Achse vorliegen. Die Konnektoren können im expandierten Zustand des Stents zur longitudinalen Achse einen Winkel bilden.

Der Grundkörper des erfindungsgemäßen Stents kann aus jedem Implantatwerkstoff bestehen, der für die Herstellung von Implantaten, insbesondere Stens, geeignet ist. Implantatwerkstoffe für Stents umfassen Polymere, metallische Werkstoffe und keramische Materialien. Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder TiA16Nb7) und Goldlegierungen. Bevorzugt weist der Grundkörper einen metallischen Implantatwerkstoff auf oder besteht daraus.

Besonders bevorzugt weist der erfindungsgemäße Stent einen Grundkörper auf, der einen biodegradierbaren Implantatwerkstoff enthält oder daraus besteht. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Insbesondere kann der Grundkörper eines erfindungsgemäßen Stents eine biokorrodierbare Magnesiumlegierung aufweisen oder daraus bestehen.

Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Die Legierungen der Elemente Magnesium, Eisen, Zink oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

In DE 197 31 021 A1 werden geeignete biokorrodierbare metallische Implantatwerkstoffe vorgestellt, deren Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stent, eignet.

Der Grundkörper des erfindungsgemäßen Stents weist mindestens einen radial asymmetrisch expandierbaren Abschnitt auf. Unter einem Abschnitt wird dabei ein Wandbereich des Grundkörpers verstanden, der sowohl eine Ausdehnung in longitudinaler Richtung als auch in radialer Richtung aufweist. Ein Abschnitt ist dann radial asymmetrisch expandierbar im Sinne der vorliegenden Erfindung, wenn dieser Abschnitt während der Expansion im Vergleich zu an den Abschnitt angrenzenden Bereichen des Grundkörpers bevorzugt gedehnt wird und damit geeignet ist, während der Expansion eine größere Kraft auf den Grundkörper umgebende Oberflächen auszuüben als die an den Abschnitt angrenzenden Bereiche des Grundkörpers.

Dazu weist der radial asymmetrisch expandierbare Abschnitt des Stents eine durchschnittliche Dehnfähigkeit auf, die erhöht ist im Vergleich zur durchschnittlichen Dehnfähigkeit im übrigen Grundkörper, insbesondere in den Bereichen des Grundkörpers, die an den radial asymmetrisch expandierbaren Abschnitt angrenzen. Unter dem Begriff Dehnfähigkeit oder Dehnbarkeit wird die Eigenschaft eines Werkstoffes verstanden, unter Krafteinwirkung seine Form zu verändern. Die Dehnfähigkeit oder Dehnbarkeit gibt an, wie weit ein Körper, ein Werkstoff oder eine Werkstoffzusammensetzung verlängert werden kann, ohne dass er bricht oder reißt. Dem Fachmann sind Mittel und Wege bekannt, um die durchschnittliche Dehnfähigkeit bestimmter Abschnitte oder Bereiche des Grundkörpers des erfindungsgemäßen Stents zu bestimmen. Da es sich erfindungsgemäß um ein relatives Verhältnis der durchschnittlichen Dehnfähigkeiten zueinander handelt, ist der Fachmann in der Wahl der Methode frei, vorausgesetzt die durchschnittliche Dehnfähigkeit im radial asymmetrisch expandierbaren Abschnitt wird mit der selben Methode gemessen wie die durchschnittliche Dehnfähigkeit im übrigen Grundkörper des Stents. Bevorzugt beträgt das Verhältnis δ der durchschnittlichen Dehnfähigkeit im übrigen Grundkörper des Stents zur durchschnittlichen Dehnfähigkeit im radial asymmetrisch expandierbaren Abschnitt 1 > δ ≥ 0,01, besonders bevorzugt 0,95 ≥ δ ≥ 0,1 besonders bevorzugt 0,9 ≥ δ ≥ 0,7.

Dabei ist zu beachten, dass die Dehnfähigkeit des asymmetrisch expandierbaren Abschnitts mit fortschreitender Dehnung abnehmen sollte (dies ist z. B. bei den oben erwähnten CoCr Legierungen der Fall) und sich das Material verfestigt. Dadurch wird ein Reißen der Streben in Bereichen erhöhter Dehnfähigkeit während der vollständigen Dilatation verhindert.

Die Dehnfähigkeit kann durch mehrere Maßnahmen beeinflusst werden. Beispielsweise kann die Dehnfähigkeit eines Abschnitts des Grundkörpers dadurch erhöht werden, dass die Bogenelemente im radial asymmetrisch expandierbaren Abschnitt eine durchschnittliche Strebenbreite aufweisen, die geringer ist als die durchschnittliche Strebenbreite der Bogenelemente im übrigen Grundkörper. Bei gleicher angewendeter Kraft dehnen sich Bogenelemente aus demselben Material und mit derselben Geometrie aber mit einer geringeren Strebenbreite vergleichsweise stärker als Bogenelemente mit größerer Strebenbreite. Bevorzugt beträgt das Verhältnis φ der durchschnittlichen Strebenbreite der Bogenelemente im radial asymmetrisch expandierbaren Abschnitt zur durchschnittlichen Strebenbreite der Bogenelemente im übrigen Grundkörper des Stents 1 ≥ φ ≥ 0,01, besonders bevorzugt 0,95 ≥ φ ≥ 0,1, besonders bevorzugt 0,9 ≥ φ ≥ 0,7.

Alternativ oder zusätzlich zur Veränderung der Strebenbreite der Bogenelemente, kann eine erhöhte Dehnfähigkeit dadurch erreicht werden, dass die durchschnittliche Maschenweite im radial asymmetrisch expandierbaren Abschnitt im Vergleich zur durchschnittlichen Maschenweite im übrigen Grundkörper erhöht ist. Eine Veränderung der Maschenweite lässt sich beispielsweise durch Veränderung der Geometrie von Konnektoren, Bogen- und/oder Diagonalelementen erreichen. Bei gleicher angewendeter Kraft dehnen sich Bereiche des Grundkörpers aus demselben Material aber mit einer größeren Maschenweite vergleichsweise stärker als Bereiche des Grundkörpers des erfindungsgemäßen Stents mit geringerer Maschenweite. Bevorzugt beträgt das Verhältnis **κ** der durchschnittlichen Maschenweite im übrigen Grundkörper des Stents zur durchschnittlichen Maschenweite im radial asymmetrisch expandierbaren Abschnitt 1 > **κ** ≧ 0,01, besonders bevorzugt 0,95 ≥ **κ** ≥ 0,1, besonders bevorzugt 0,9 ≥ **κ** ≥ 0,7.

Alternativ oder zusätzlich kann im erfindungsgemäßen Stent eine erhöhte Dehnfähigkeit dadurch erreicht werden, dass der durchschnittliche Abstand der aufeinander folgenden umlaufenden Stützstrukturen im radial asymmetrisch expandierbaren Abschnitt größer ist als der durchschnittliche Abstand der aufeinander folgenden umlaufenden Stützstrukturen im übrigen Grundgerüsts des Stents. Ein größerer Abstand der Stützstrukturen voneinander kann beispielsweise dadurch erreicht werden, dass die Geometrie der Konnektoren beeinflusst wird. Bevorzugt kann dies dadurch erreicht werden, dass die durchschnittliche Länge der Konnektoren im radial asymmetrisch expandierbaren Abschnitt größer ist als die durchschnittliche Länge der Konnektoren im übrigen Grundkörper des erfindungsgemäßen Stents. Bei gleicher angewendeter Kraft dehnen sich Bereiche des Grundkörpers aus dem selben Material und mit der selben Geometrie aber mit einem größeren Abstand zwischen den aufeinander folgenden Stützstrukturen vergleichsweise stärker als Bereiche des Grundkörpers des erfindungsgemäßen Stents mit geringerem Abstand zwischen den Stützstrukturen. Bevorzugt beträgt das Verhältnis **ε** des durchschnittlichen Abstands der aufeinander folgenden umlaufenden Stützstrukturen im übrigen Grundgerüsts des Stents zum durchschnittlichen Abstand der aufeinander folgenden umlaufenden Stützstrukturen im radial asymmetrisch expandierbaren Abschnitt 1 > **ε** ≥ 0,01, besonders bevorzugt 0,95 ≥ **ε** ≥ 0,1, besonders bevorzugt 0,9 ≥ **ε** ≥ 0,7.

Die Form, Positionierung und Anzahl von radial asymmetrisch dilatierbaren Abschnitten des erfindungsgemäßen Stents ist grundsätzlich frei wählbar und kann sich beispielsweise im Wesentlichen nach der Form, der Lage und der Ausdehnung des zu behandelnden Areals im Gefäß richten.

Der erfindungsgemäße Stent kann beispielsweise lediglich einen einzigen radial asymmetrisch dilatierbaren Abschnitt aufweisen.

Der erfindungsgemäße Stent kann auch mehrere getrennt voneinander vorliegende, aufeinander folgende oder überlappende radial asymmetrisch dilatierbare Abschnitte aufweisen. Die radial asymmetrisch dilatierbaren Abschnitte können in einer bestimmten geometrischen Form gestaltet sein, sie können beispielsweise unabhängig voneinander kreisförmig, elliptisch oder streifenförmig ausgebildet sein.

Der erfindungsgemäße Stent kann sich beispielsweise dadurch auszeichnen, dass er einen einzigen radial asymmetrisch dilatierbaren Abschnitt aufweist und dieser sich im Wesentlichen über den gesamten expandierbaren Grundkörper erstreckt.

Weist der erfindungsgemäße Stent mehr als einen radial asymmetrisch dilatierbaren Abschnitt auf, so kann diese Mehrzahl derart ausgeführt und angeordnet sein, dass die Summe der Längsausdehnungen der einzelnen radial asymmetrisch dilatierbaren Abschnitte nicht mehr als 50% der Längsausdehnung des expandierbaren Grundkörpers des Stents beträgt.

Das Vorhandensein mehrerer radial asymmetrisch dilatierbaren Abschnitte auf einem erfindungsgemäßen Stent kann bei geeigneter Ausgestaltung und Positionierung beispielsweise auch dazu dienen eine unerwünschte Lageänderung des Stents während der Expansion zu verhindern. Insbesondere können die zu den therapeutisch eingesetzten radial asymmetrisch dilatierbaren Abschnitten zusätzlich vorhandenen radial asymmetrisch dilatierbaren Abschnitte derart ausgerichtet und ausgestaltet sein, dass eine ungewollte Rotationsbewegung des Stents während der Expansion im Gefäß vermieden oder mindestens erschwert wird. Zu einer solchen Lageänderung kann es beispielsweise dann kommen, wenn der radial asymmetrisch dilatierbaren Abschnitt auf die zu dehnende Läsion trifft und von dieser abgelenkt wird oder von ihr abrutscht bevor der Rest des Stents ausreichend aufgeweitet wurde um eine stabile Lage im Gefäß sicherzustellen und damit eine ungewollte Rotationsbewegung im Gefäß zu unterbinden.

Die vorliegende Erfindung bezieht sich auch auf einen Katheter zur Applikation des erfindungsgemäßen Stents, wobei der erfindungsgemäße Stent auf den Katheter gecrimpt vorliegt. Geeignete Katheter sind dem Fachmann bekannt.

Um die Vorteile des erfindungsgemäßen Stents besonders effektiv nutzen zu können, ist es sinnvoll, dafür zu sorgen, dass der Stent vor der Expansion am gewünschten Ort im Gefäß derart radial ausgerichtet ist, dass der radial asymmetrisch expandierbare Abschnitt auch auf der zu behandelnden Stelle zu liegen kommt und nicht auf umliegendem gesundem Gewebe.

Für den Erfolg der asymmetrischen Kraftverteilung durch den erfindungsgemäßen Stent ist es also wünschenswert, dass der Stent innerhalb des Gefäßes exakt positionierbar ist. Dabei muss nicht nur die longitudinale Position gewährleistet werden, sondern auch der Stent durch Rotation in die richtige radiale Ausrichtung gebracht werden.

Dazu kann der erfindungsgemäße Katheter mit Mitteln oder Markern versehen sein, die eine exakte Positionierung des Katheters innerhalb eines Gefäßes erlauben. Dem Fachmann ist dabei bekannt, das außer der unten beschriebenen Methode, weitere Methoden und bildgebende Verfahren existieren, um die exakte Positionierung eines Katheters innerhalb des Gefäßes zu erzielen. So kann z. B. mit Hilfe von intravaskulären Bildgebungsverfahren (Ultraschall, optische Kohäreztomographie) in Kombination mit einem Katheter, dieser exakt innerhalb des Gefäßes ausgerichtet werden. Weiterhin gibt es Verfahren, die die räumliche Lage eines Katheters innerhalb eines Lumens unter Zuhilfenahme von Permanentmagneten in der Katheterspitze bestimmen.

Für ein einfacheres Verfahren kann folgendes Prinzip angewendet werden:
Die Oberfläche des Innenschafts und/oder Außenschafts des Katheters kann einen oder mehrere Marker aufweisen, die derart angeordnet und orientiert sind, dass während der Applikation des Katheters im Gefäß des zu behandelnden Individuums die räumliche Ausrichtung des radial asymmetrisch dilatierbaren Abschnitts des erfindungsgemäßen Stents bestimmbar ist. Bevorzugt sind die Marker auf dem Katheter derart positioniert und gruppiert, dass diese vom gecrimpten, erfindungsgemäßen Stent nicht überdeckt werden und die Rotationsstellung des radial asymmetrisch dilatierbaren Abschnitts des Stents im Gefäß bestimmbar ist.

Dabei werden bevorzugt Marker eingesetzt, die sich von außerhalb des Körpers mittels bildgebender Verfahren erfassen und darstellen lassen. Dem Fachmann sind geeignete Marker bekannt. Beispielsweise kann es sich um bekannte Röntgen- und/oder Fluoreszenzmarker handeln. Um eine sichere Detektion und Darstellung der dreidimensionalen Lage des Katheters im Gefäß zu erlauben, werden bevorzugt mehr als eine Markersubstanz bzw. mehr als ein Marker eingesetzt, besonders bevorzugt mindestens zwei verschiedene Marker, ganz besonders bevorzugt mindestens drei verschiedene Marker. Durch den Einsatz mehrerer verschiedener Marker wird das Risiko von Artefakten in der Bildgebung verringert.

Dabei werden die Marker bevorzugt in unterschiedlichen geometrischen Figuren auf der Oberfläche des Innenschafts und/oder Außenschafts des Katheters angeordnet. Bei den geometrischen Figuren handelt es sich bevorzugt um Ringe, Bänder, Dreiecke und/oder Pfeile, wobei die erstgenannten als Referenzsysteme zur späteren Bildverarbeitung dienen. Diese geometrischen Figuren können dabei derart gestaltet werden, dass trotz einer zweidimensionalen Bildgebung (in einer Art "Draufsicht") die Orientierung des Katheters im dreidimensionalen Raum bestimmbar ist. Dazu können verschiedene Figuren miteinander kombiniert werden. Besonders vorteilhaft ist die Verwendung von Dreiecken, die sich insbesondere über den halben Katheterumfang erstrecken können. In einer bevorzugten Ausführungsform werden mindestens drei gleiche oder verschiedene geometrische Figuren derart auf der Oberfläche des Katheters angebracht und ausgerichtet, so dass die Rotationsstellung des oder der radial asymmetrisch dilatierbaren Abschnitte des darauf gecrimpten erfindungsgemäßen Stents bestimmbar ist.

Bevorzugt werden mindestens zwei Dreiecke eingesetzt, deren Spitzen gegenläufig angeordnet sind. Besonders bevorzugt werden drei Dreiecke verwendet, deren Spitzen alternierend gegenläufig zueinander angeordnet sind und deren Basis jeweils derart zueinander angeordnet ist, dass ein für jede Rotationslage des Katheters spezifisches, eindeutiges zweidimensionales Bild erhalten wird. Dieses Bild kann mit Hilfe von Bildanalyseverfahren automatisch analysiert werden und direkt in eine Rotationsposition umgerechnet werden. Dabei dienen die Radiomarker an den Enden des Ballons als Referenzsysteme, die es erlauben, sowohl die Fläche als auch die geometrische Form (in ihrer zweidimensionaler Darstellung) der dreieckigen Positionsmarkierungen eindeutig zu bestimmen.

Figuren:

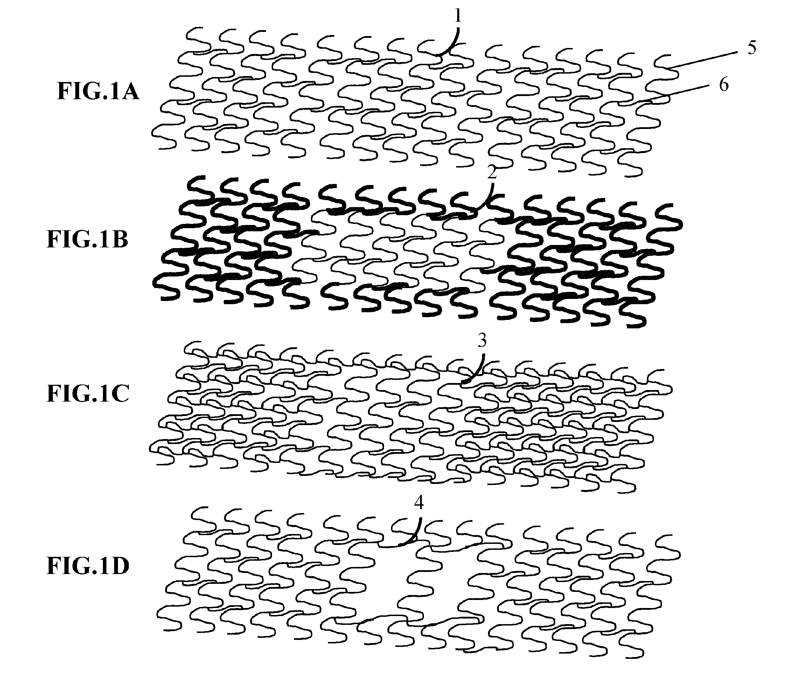

- Figur 1: zeigt eine schematische Darstellung eines Ausschnitts aus einem Grundkörper eines Stents, wobei in (**A**) ein Stent nach Stand der Technik gezeigt ist, während in (**B**), (**C**) und (**D**) jeweils unterschiedliche Ausführungsformen eines erfindungsgemäßen Stents mit einem radial asymmetrisch expandierbaren Abschnitt dargestellt sind.

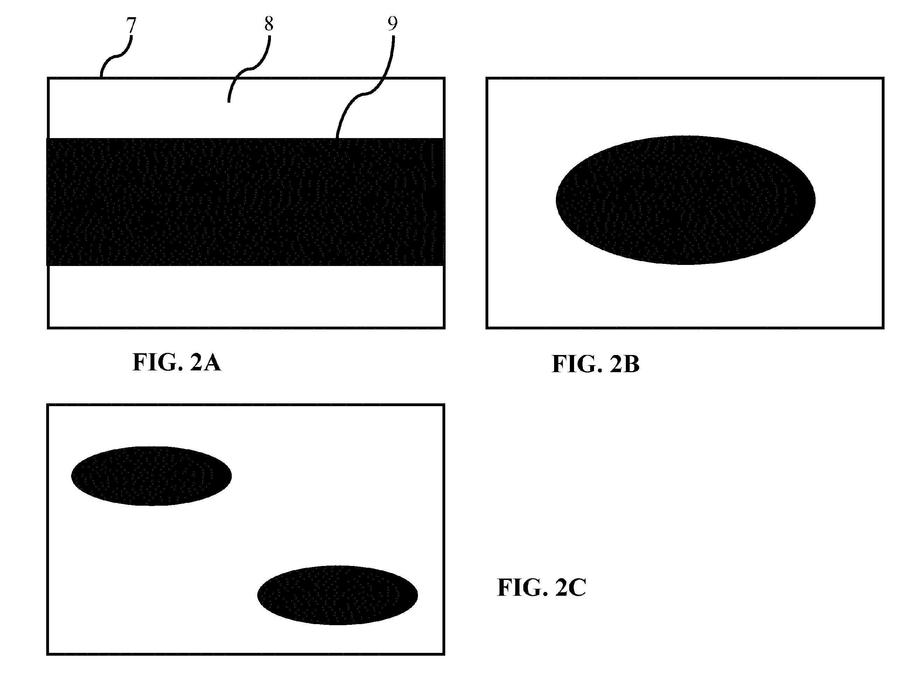

- Figur 2: zeigt eine schematische Darstellung der Gestaltung von radial asymmetrisch dilatierbaren Abschnitten eines erfindungsgemäßen Stents. (A) Darstellung eines radial asymmetrisch dilatierbaren Abschnitts in Streifenform; (B) Darstellung eines radial asymmetrisch dilatierbaren Abschnitts in Ellipsenform; (C) Darstellung eines Ausschnitts eines Stents mit zwei ellipsenförmigen radial asymmetrisch dilatierbaren Abschnitten.

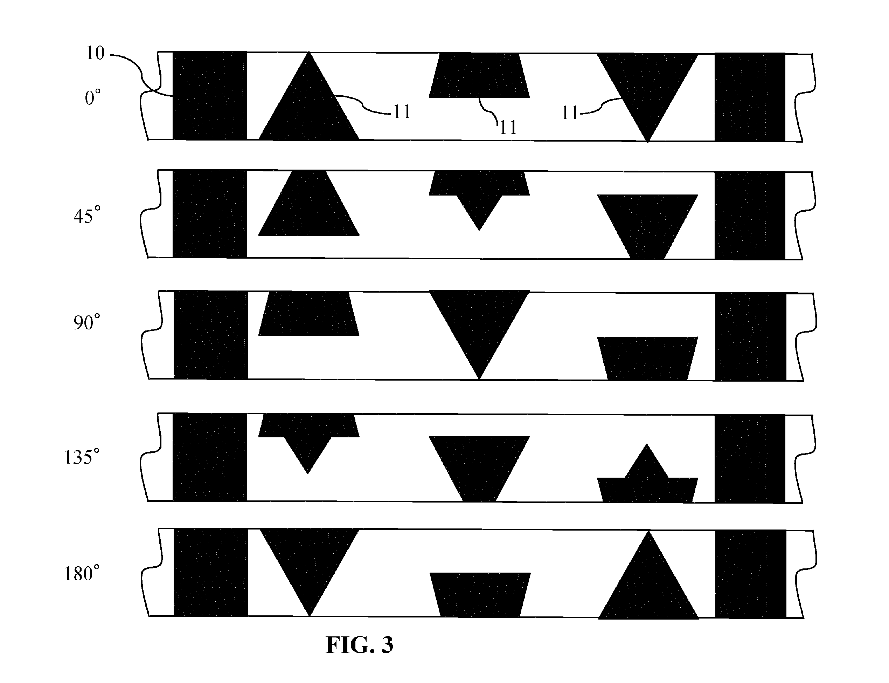

- Figur 3: zeigt eine zweidimensionale Darstellung (als "Draufsicht") eines zum erfindungsgemäßen Stent gehörenden Katheters mit auf der Oberfläche angeordneten Markern in unterschiedlichen Stadien der Rotation.

Nachfolgend wird die Erfindung anhand von Ausführungsbeispielen näher erläutert.

### Ausführungsbeispiel 1

In Figur 1A ist ein Bereich eines Grundkörpers eines herkömmlichen Stents gezeigt. In einem Ausschnitt 1 ist gezeigt, dass der Grundkörper aus einer regelmäßigen Struktur von Stützstrukturen 5 und Konnektoren 6 gebildet ist. Die dargestellten Bereiche des Grundkörpers weisen eine einheitliche durchschnittliche Dehnfähigkeit auf. Alle Elemente weisen im Wesentlichen die gleiche Geometrie, Strebenbreite und Maschenweite auf, so dass der Grundkörper dieses Stents eine radial symmetrische Kraftverteilung während der Expansion des Stents zeigt.

In Figur 1B ist ein Ausschnitt aus einer ersten Ausführungsform eines erfindungsgemäßen Stents gezeigt, der einen radial asymmetrisch expandierbaren Abschnitt 2 aufweist. Der radial asymmetrisch expandierbare Abschnitt 2 zeichnet sich dadurch aus, dass die durchschnittliche Strebenbreite der Bogenelemente der Stützstrukturen verringert ist im Vergleich zur durchschnittlichen Strebenbreite der Bogenelemente der Stützstrukturen die an den radial asymmetrisch expandierbaren Abschnitt angrenzen. Dadurch wird die Dehnfähigkeit erhöht und es wird im Abschnitt 2 eine bevorzugte Dehnung während der Expansion des Stents ermöglicht. Um den Effekt auf die Dehnfähigkeit noch zu erhöhen, kann die durchschnittliche Strebenbreite nicht nur der Bogenelemente sondern zusätzlich auch die durchschnittliche Strebenbreite der Diagonalelemente und/oder der Konnektoren verringert sein im Vergleich zur durchschnittlichen Strebenbreite der entsprechenden Strukturen im übrigen Grundgerüst.

In Figur 1C ist ein Ausschnitt aus einer zweiten Ausführungsform eines erfindungsgemäßen Stents gezeigt, der einen radial asymmetrisch expandierbaren Abschnitt 3 aufweist. Der radial asymmetrisch expandierbare Abschnitt 3 zeichnet sich dadurch aus, dass Konnektoren mit einer Geometrie verwendet werden, die sich von der Geometrie der Konnektoren in den übrigen, den Abschnitt 3 umgebenden Bereichen des Grundkörpers derart unterscheidet, dass der radial asymmetrisch expandierbare Abschnitt 3 eine erhöhte Dehnfähigkeit aufweist im Vergleich zur Dehnfähigkeit der übrigen, den Abschnitt 3 umgebenden Bereiche des Grundkörpers.

In Figur 1D ist ein Ausschnitt aus einer dritten Ausführungsform eines erfindungsgemäßen Stents gezeigt, der einen radial asymmetrisch expandierbaren Abschnitt 4 aufweist. Der radial asymmetrisch expandierbare Abschnitt 4 zeichnet sich dadurch aus, dass im asymmetrisch expandierbaren Abschnitt 4 der Abstand der aufeinander folgenden Stützstrukturen voneinander vergrößert ist, in dem Konnektoren verwendet werden, die länger sind als die Konnektoren in den übrigen den Abschnitt 4 umgebenden Bereichen des Grundkörpers. Dadurch weist der Grundkörper im Abschnitt 4 eine erhöhte Dehnfähigkeit auf im Vergleich zur Dehnfähigkeit der übrigen den Abschnitt 4 umgebenden Bereiche des Grundkörpers.

### Ausführungsbeispiel 2

In Figur 2 sind ausgewählte Gestaltungen von radial asymmetrisch dilatierbaren Abschnitten eines erfindungsgemäßen Stents schematisch dargestellt. In Figur 2A ist ein Ausschnitt des Grundkörpers 7 eines erfindungsgemäßen Stents gezeigt mit einem radial asymmetrisch dilatierbaren Abschnitt 9 in Streifenform. Dabei erstreckt sich der radial asymmetrisch dilatierbaren Abschnitt 9 der Längsachse des Ausschnitts des Grundkörpers 7. Der radial asymmetrisch dilatierbaren Abschnitt 9 wird seitlich von einem Bereich 8 begrenzt, der eine im Vergleich zum radial asymmetrisch dilatierbaren Abschnitt 9 eine geringere Dehnfähigkeit aufweist.

In Figur 2B ist ein radial asymmetrisch dilatierbarer Abschnitt schematisch dargestellt, der eine Ellipsenform aufweist.

In Figur 2C ist ein Ausschnitt eines Grundkörpers gezeigt, der zwei radial asymmetrisch dilatierbarer Abschnitte aufweist, wobei in diesem Fall beide radial asymmetrisch dilatierbare Abschnitte exemplarisch in Ellipsenform gezeigt sind. Die radial asymmetrisch dilatierbaren Abschnitte können natürlich auch andere Formen annehmen.

### Ausführungsbeispiel 3

In Figur 3 ist eine Anordnung von Markern auf der Oberfläche eines erfindungsgemäßen Katheters zur Applikation eines erfindungsgemäßen Stents schematisch dargestellt, wobei die Marker derart positioniert und angeordnet sind, dass die Rotationslage des Katheters im Gefäß auch in einer zweidimensionalen Darstellung (als "Draufsicht") eindeutig bestimmbar ist. Der Katheter weist auf seiner Oberfläche Marker in Form von zwei Bändern 10 und mindestens einem Dreieck 11, bevorzugt zwei Dreiecken 11, besonders bevorzugt drei Dreiecken 11 auf.

Dabei sind die drei Dreiecke 11 derart orientiert und aufeinander abgestimmt, dass sich aus deren zweidimensionalem Bild exakt und eindeutig die Rotationslage des Katheters im Gefäß bestimmen lässt. Ist die Rotationslage des Katheters bekannt, kann auch die Lage des radial asymmetrisch expandierbaren Abschnitts des auf dem Katheter gecrimpt vorliegenden, erfindungsgemäßen Stents bestimmt werden. Dazu sind die drei Dreiecke 11 in ihrer Ausrichtung der Spitze alternierend angeordnet. Die Dreiecke 11 erstrecken sich in ihrer Ausdehnung von der Spitze bis zur Basis im Wesentlichen jeweils über den halben Umfang des Katheters. Wie in Figur 3 gezeigt, ergibt sich aus dieser Anordnung der Dreiecke 11 für jede Rotationslage (0° bis 180°) ein eindeutiges zweidimensionales Bild, so dass die Bestimmung der Rotationslage anhand von erhaltenen zweidimensionalen Bildern bestimmt werden kann. Dabei ist es ausreichend, wenn mindestens ein Dreieck 11 abgebildet werden kann. Um allerdings den Effekt von Artefaktbildung während der Bildgebung zu minimieren, kann es sinnvoll sein, mehrere dieser Dreiecke 11 in unterschiedlicher Rotation und Orientierung zueinander zu verwenden, wobei zwei Dreiecke die selbe Orientierung aber unterschiedliche Rotationslagen aufweisen können. Bevorzugt werden Röntgenmarker, Fluoreszenzmarker und/oder Kombinationen daraus eingesetzt.

## Patentansprüche

1. Expandierbarer Stent umfassend einen röhrenförmigen Grundkörper mit einem Lumen entlang einer longitudinalen Achse, **dadurch gekennzeichnet, dass** der Grundkörper mindestens einen radial asymmetrisch expandierbaren Abschnitt aufweist, wobei der radial asymmetrisch expandierbare Abschnitt eine durchschnittliche Dehnfähigkeit aufweist, die im Vergleich zur durchschnittlichen Dehnfähigkeit im übrigen Grundkörper des Stents erhöht ist.

2. Stent nach Anspruch 1, **dadurch gekennzeichnet, dass** das Verhältnis δ der durchschnittlichen Dehnfähigkeit im übrigen Grundkörper des Stents zur durchschnittlichen Dehnfähigkeit im radial asymmetrisch expandierbaren Abschnitt 1 > **δ** ≥ 0,01 beträgt, bevorzugt 0,95 ≥ **δ** ≥ 0,1 und besonders bevorzugt 0,9 ≥ **δ** ≥ 0,7.

3. Stent nach einem der vorhergehenden Ansprüche, **dadurch gekennzeichnet, dass** der Grundkörper des Stents eine Mehrzahl von umlaufenden Stützstrukturen aufweist, die aufeinander folgend entlang der longitudinalen Achse angeordnet sind und jeweils aus einer radialen Aneinanderreihung von Diagonalelementen und Bogenelementen aufgebaut sind; und der Grundkörper einen oder mehrere Konnektoren aufweist, wobei zwei aufeinander folgende umlaufende Stützstrukturen über mindestens einen Konnektor miteinander verbunden vorliegen.

4. Stent nach Anspruch 3, **dadurch gekennzeichnet, dass** die Bogenelemente im radial asymmetrisch expandierbaren Abschnitt eine durchschnittliche Strebenbreite aufweisen, die geringer ist als die durchschnittliche Strebenbreite der Bogenelemente im übrigen Grundkörper.

5. Stent nach Anspruch 4, **dadurch gekennzeichnet, dass** das Verhältnis φ der durchschnittlichen Strebenbreite der Bogenelemente im radial asymmetrisch expandierbaren Abschnitt zur durchschnittlichen Strebenbreite der Bogenelemente im übrigen Grundkörper des Stents 1 > **φ** ≥ 0,01 beträgt, bevorzugt 0,95 ≥ **φ** ≥ 0,1 und besonders bevorzugt 0,9 ≥ **φ** ≥ 0,7.

6. Stent nach einem der Ansprüche 3 bis 5, **dadurch gekennzeichnet, dass** die durchschnittliche Maschenweite im radial asymmetrisch expandierbaren Abschnitt im Vergleich zur durchschnittlichen Maschenweite im übrigen Grundkörper erhöht ist.

7. Stent nach Anspruch 6 **dadurch gekennzeichnet, dass** das Verhältnis **κ** der durchschnittlichen Maschenweite im übrigen Grundkörper des Stents zur durchschnittlichen Maschenweite im radial asymmetrisch expandierbaren Abschnitt 1 > **κ** ≥ 0,01 beträgt, bevorzugt 0,95 ≥ **κ** ≥ 0,1 und besonders bevorzugt 0,9 ≥ **κ** ≥ 0,7.

8. Stent nach einem der Ansprüche 3 bis 7, **dadurch gekennzeichnet, dass** der durchschnittliche Abstand der aufeinander folgenden umlaufenden Stützstrukturen im radial asymmetrisch expandierbaren Abschnitt größer ist als der durchschnittliche Abstand der aufeinander folgenden umlaufenden Stützstrukturen im übrigen Grundgerüsts des Stents.

9. Stent nach Anspruch 8, **dadurch gekennzeichnet, dass** das Verhältnis **ε** des durchschnittlichen Abstands der aufeinander folgenden umlaufenden Stützstrukturen im übrigen Grundgerüsts des Stents zum durchschnittlichen Abstands der aufeinander folgenden umlaufenden Stützstrukturen im radial asymmetrisch expandierbaren Abschnitt 1 > **ε** ≥ 0,01 beträgt, bevorzugt 0,95 ≥ **ε** ≥ 0,1 und besonders bevorzugt 0,9 ≥ **ε** ≥ 0,7.

10. Katheter zur Applikation eines Stents nach einem der Ansprüche 1 bis 9, wobei der Stent auf dem Katheter angeordnet ist.

11. Katheter nach Anspruch 10, **dadurch gekennzeichnet, dass** die Oberfläche des Katheters einen oder mehrere Marker aufweist, die derart angebracht und orientiert sind, dass während der Positionierung des Katheters in einem Gefäß des zu behandelnden Individuums die räumliche Ausrichtung des radial asymmetrisch dilatierbaren Abschnitts des Stents bestimmbar ist, bevorzugt ist die Rotationsstellung des radial asymmetrisch dilatierbaren Abschnitts des Stents bestimmbar.

12. Katheter nach Anspruch 11, **dadurch gekennzeichnet, dass** es sich bei den Markern um Substanzen handelt, die durch bildgebende Verfahren detektierbar und/oder darstellbar sind, bevorzugt handelt es sich um Röntgen- und/oder Fluoreszenzmarker.

13. Katheter nach einem der Ansprüche 11 bis 12, **dadurch gekennzeichnet, dass** die Marker in unterschiedlichen oder gleichen geometrischen Figuren auf der Oberfläche des Katheterschafts angeordnet sind.

14. Katheter nach Anspruch 13, **dadurch gekennzeichnet, dass** die geometrischen Figuren Ringe, Bänder, Dreiecke und/oder Pfeile umfassen oder daraus bestehen.

15. Katheter nach Anspruch 13 oder 14, **dadurch gekennzeichnet, dass** mindestens drei gleiche oder verschiedene geometrische Figuren derart auf der Oberfläche des Katheterschafts ausgerichtet sind, so dass die Rotationsstellung des oder der radial asymmetrisch dilatierbaren Abschnitte bestimmbar ist.
